Europäisches Patentamt

European Patent Office

Office européen des brevets

(19)

(11) Publication number: **0 039 878**
**B1**

(12) **EUROPEAN PATENT SPECIFICATION**

(45) Date of publication of patent specification: **28.12.83**

(51) Int. Cl.³: **C 07 C  69/80,  C 07 C  67/08**

(21) Application number: **81103376.0**

(22) Date of filing: **05.05.81**

(54) **Method of making mixed esters of phthalic acid.**

(30) Priority: **08.05.80 US  147761**

(43) Date of publication of application:
**18.11.81 Bulletin 81/46**

(45) Publication of the grant of the patent:
**28.12.83 Bulletin 83/52**

(84) Designated Contracting States:
**AT BE DE FR GB IT NL**

(56) References cited:
**GB - A - 654 842**
**GB - A - 1 310 114**
**US - A - 2 661 367**

(73) Proprietor: **BASF WYANDOTTE CORPORATION**
**1609 Biddle Avenue**
**Wyandotte Michigan 48192 (US)**

(72) Inventor: **Evangelista, John Joseph**
**8 Cornell Drive**
**Succasunna New Jersey 07876 (US)**
Inventor: **Deck, Charles Francis**
**2805 Trenton Drive**
**Trenton Michigan 48183 (US)**

(74) Representative: **Michaelis, Wolfgang, Dr. et al,**
**c/o BASF Aktiengesellschaft 38 Carl-Bosch-**
**Strasse**
**D-6700 Ludwigshafen (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Method of making mixed esters of phthalic acid

This invention relates to a new method of preparing mixed esters of phthalic acid for use as plasticizers particularly for vinyl plastics and more specifically, mixed esters from phthalic anhydride, butyl alcohol, and octyl alcohol by sequential reaction.

A plasticizer has been defined as a material incorporated in a plastic to increase its workability and its flexibility and distensibility. The addition of the plasticizer may lower the melt viscosity, the temperature of the second-order transition, or the elastic modulus of the plastic. A class of such plasticizers, which has been widely used, is the phthalic acid esters of monoalcohols produced by condensing aliphatic monoalcohols containing not more than 20 carbon atoms with phthalic anhydride to form neutral products. Dioctylphthalate, known as DOP, is the industry standard for a general purpose plasticizer for poly (vinyl chloride) (PVC) and accounts for approximately one-fourth of the total plasticizer production. Since plasticization of poly (vinyl chloride) takes an estimated 80 percent of plasticizer production, most plasticizers are compared to DOP for price/performance relationships. The economic life of any other plasticizer depends upon its being less expensive than DOP or its doing some specific job better than DOP. The demand from the public in the last decade for high-quality products has resulted in continued growth of many different plasticizers with unique properties. Among the better known plasticizers for PVC are the co-esters of butanol and octanol with phthalic anhydride generally referred to as butyl octyl phthalate (BOP) which have been available in the U.S. market since the early 1950's and butyl benzyl phthalate (BBP). It must be understood that the products referred to on the market as butyl octyl phthalate are not necessarily completely butyl octyl phthalate. Generally, when made by the mixed ester route, the final product is a ternary mixture of dibutyl phthalate, butyl octyl phthalate and dioctyl phthalate. The ratio of these three components can vary widely, based not only upon the stoichiometry of the original reaction, but also upon the manufacturing procedure and catalyst employed. Generally, the prior art butyl octyl phthalate products tend to have around a 20/80 mole percent ratio of $C_4$ to $C_8$ alcohol residues.

During the 1950's, butyl octyl phthalate and butyl benzyl phthalate sold at a price of about one-half cent a pound below that of DOP. As such, these two plasticizers represented low-priced replacements for DOP. In the mid-1960's, both butyl octyl phthalate and butyl benzyl phthalate took the form of specialty plasticizers and the price rose above that of DOP. The specialty aspect emphasized was high solvency. This characteristic is particularly valuable in the manufacture of products such as vinyl asbestos tile, carpet backing, plastisols and organosols, coatings and as process aids. The latter, particularly, involve the addition of plasticizers to formulations used in calendering and extrusion where the primary performance characteristic is to reduce apparent melt viscosity at processing temperature and/or to provide improved surface gloss.

However, both products have the drawback of high volatility. In the case of the butyl octyl phthalate, this is due primarily to the presence of dibutyl phthalate.

Accordingly, it is desirable to produce a higher solvating plasticizer than DOP with reduced volatility compared to BBP to prior art butyloctylphthalate products by producing a butyloctylphthalate product have an improved BOP/DOP ratio and a minimum dibutylphthalate (DBP) content.

A substantial improvement in this direction has been achieved by the invention set fourth in U.S. Patent Application Serial No. 069,636, filed August 27, 1979 corresponding to EP.A O. 024505. This application discloses reacting butyl alcohol with phthalic anhydride to provide a mixture containing the half-ester and unreacted phthalic anhydride. This first step requires initial heating but exotherms near 90°C and it is necessary to maintain the temperature below 130°C. This reaction generally requires about 50 to 60 minutes. The reaction mixture is then cooled and octylalcohol is added along with a catalyst. The mixture is then heated with agitation to a temperature of about 160°C at which point it refluxes. It is allowed to rise slowly to a temperature of about 200°C and is not permitted to exceed 210°C. The reaction is continued until the water ceases to separate in the distillate.

While this is a substantial improvement over the prior art, it is a purpose of the present invention to effect a substantial further improvement in the production of a butyloctylphthalate product, i.e., to further and substantially improve the combination of BOP/DOP ratio and dibutylphthalate concentration.

The present invention is directed to an improvement in the process for preparing butyloctylphthalate products which are mixed esters of phthalic acid for use as plasticizers characterized by improved solvating characteristics and reduced volatility. More specifically, the present invention is an improvement in the process of U.S. Patent Application Serial No. 069,636 wherein the second step reaction temperature is maintained at a maximum of about 150°C and preferably about 140°C, and wherein vacuum is applied in amounts sufficient to maintain reflux. It was found that butyloctylphthalate products having a greatly improved combination of BOP/DOP ratio and dibutylphthalate content was achieved, whereas in the case of butyloctyl-phthalate esters obtained by prior art methods the BOP/DOP ratio is about 0.5 or less and the DBP content is about 8, the butyloctyl-phthalate esters obtained according to the present invention (see the Examples) have a BOP/DOP ratio of about 1 or more and a DBP content of about 7 or less.

The present invention is directed to the process where an alcohol composition consisting of 100 to 80 mole percent butyl alcohol and 0 to 20 mole percent octyl alcohol is reacted with phthalic anhydride in a mole ratio of about 0.80 to 1.5 moles alcohol total, i.e., butyl alcohol plus octyl alcohol, per mole of phthalic anhydride. The butyl alcohols that may be employed include isobutyl alcohol and n-butyl alcohol. The octyl alcohols that may be employed include isooctyl alcohol and 2-ethylhexyl alcohol. The alcohol-phthalic anhydride reaction requires initial heating, generally to about 80°C and exotherms near 90°C, and it is necessary to maintain the temperature below about 120°C. The reaction generally requires about 0.25 to 3 hours. Octyl alcohol is then added in a stoichiometric amount based on unreacted acid groups plus 20 to 100 percent of the stoichiometric amount of octyl alcohol as excess along with a catalyst. The mixture is heated gradually with agitation to a maximum temperature of about 150°C, preferably about 140°C. In general, the temperature should be at least about 120°C. Vacuum is applied to maintain boilup (as much as necessary often <10 millimeters Hg absolute pressure) and the reactor overheads are fractionated (in a column or partial condenser or suitably designed decanter) so as to reflux octanol back to the reactor while removing water of reaction together with liberated butanol. The reaction is continued until the water ceases to separate in the distillate. When the acid number drops to 2—3, the reaction is considered complete. The reaction time generally runs from about 1 to 10 hours depending on many factors such as the size of the reactor. When the reaction is complete, the mixture is cooled and the acid neutralized with an alkali such as sodium hydroxide in a sufficient amount determined by acid numbers to completely neutralize the acid plus 10 percent excess. The ester is washed by adding water and salt, stirred for about 10 to 40 minutes, allowed to settle and the aqueous layer drawn off. It is then rewashed with water and salt mixture. The system is then closed and subjected to a vacuum from about 15 to 25 inches of mercury absolute after which the alcohol is distilled off. After most of the alcohol is boiled off, the ester is stripped with steam until the free octyl alcohol content is less than 150 parts per million. After stripping, the ester is further purified by adding powdered carbon, filter aid, attapulgus clay and filtering.

Suitable catalysts which may be employed in the second step of the reaction, i.e., the reaction of the half-ester mixture with the octyl alcohol, include tetra-n-butyl titanate, a tin-titanate blend, and strong mineral acids such as sulfuric. Other catalysts that may be employed are p-toluenesulfonic acid, methanesulfonic acid, stannous octoate, and orthophosphoric acid. Alkaline solutions which may be employed for neutralizing include aqueous sodium hydroxide, aqueous potassium hydroxide, sodium bicarbonate, and calcium hydroxide.

The following examples illustrate the invention. All parts are by weight and all temperatures are in degrees Celsius unless otherwise indicated.

## Example 1

A mixture of phthalic anhydride and n-butanol was prepared in a 2-liter round bottom flask fitted with a heating mantle, temperature controller, stripping column, Stark & Dean trap, vacuum gauge, vacuum pump and variable speed agitator, by mixing 385.1 grams (2.6 moles) phthalic anhydride with 192.7 grams (2.6 moles) butanol. The mixture was heated and maintained at 90 to 95°C for 2 hours. A second alcohol charge was then made consisting of 677.2 grams (5.2 moles) of 2-ethylhexanol. Catalyst was then added (2.1 grams p-toluenesulfonic acid dissolved in 2 milliliters distilled water) and the batch heated and maintained at 120°C for 3 hours under a total pressure of 100 millimeters of mercury absolute. None of the overhead vapor was returned to the pot as condensate. The batch was then heated and maintained at 140°C for an additional 2.5 hours under total pressure of 200 millimeters mercury. The resulting mercury product analyzed as follows.

|  | Percent by Weight |
| --- | --- |
| n-butanol | 2.55 |
| 2-ethylhexanol | 17.62 |
| monobutylphthalate | 0.31 |
| mono-2-ethylhexylphthalate | 1.51 |
| dibutylphthalate | 2.86 |
| butyl-2-ethylhexylphthalate | 45.17 |
| di-2-ethylhexylphthalate | 29.78 |

The BOP/DOP weight ratio was 1.52 and the amount of dibutylphthalate as percent of total diester was 3.68.

3

# 0 039 878

### Example 2

A mixture of phthalic anhydride, n-butanol and 2-ethylhexanol was prepared in a 2-liter round bottom flask fitted with a heating mantle, temperature controller, stripping column, Stark & Dean trap, vacuum gauge, vacuum pump and variable speed agitator, by mixing 385.1 grams (2.6 moles) phthalic anhydride with 173.4 grams (2.34 moles) butanol, and 33.9 grams (0.26 moles) of 2-ethylhexanol. The mixture was heated and maintained at 90 to 95°C for 2 hours. A second alcohol charge was then made consisting of 609.5 grams (4.68 moles) of 2-ethylhexanol. Catalyst was then added (2.1 grams p-toluenesulfonic acid dissolved in 2 milliliters distilled water) and the batch heated and maintained at 130 to 135°C for 3 hours under a total pressure of 100 millimeters of mercury absolute. The resulting product analyzed as follows.

|  | Percent by Weight |
|---|---|
| n-butanol | 0.40 |
| 2-ethylhexanol | 11.60 |
| monobutylphthalate | 0.28 |
| mono-2-ethylhexylphthalate | 5.77 |
| dibutylphthalate | 2.50 |
| butyl-2-ethylhexylphthalate | 42.61 |
| di-2-ethylhexylphthalate | 37.09 |

The BOP/DOP weight ratio was 1.15 and the amount of dibutylphthalate as percent of total diester was 2.51.

### Example 3

A mixture of phthalic anhydride, n-butanol and 2-ethylhexanol was prepared in a 2-liter round bottom flask fitted with a heating mantle, temperature controller, stripping column, Stark & Dean trap, vacuum gauge, vaccum pump and variable speed agitator, by mixing 370.3 grams (2.5 moles) phthalic anhydride with 166.8 grams (2.25 moles) butanol and 32.6 grams (0.25 moles) 2-ethylhexanol. The mixture was heated and maintained at 110°C for 35 minutes. A second alcohol charge was then made consisting of 520.9 grams (4.0 moles) 2-ethylhexanol. Catalyst was then added (2.0 grams p-toluenesulfonic acid dissolved in 2.0 milliliters distilled water) and the batch heated and maintained at 140°C for 5 hours. Total pressure decreased from 760 to 100 millimeters of mercury absolute during the reaction. The resulting product analyzed as follows on a mole basis:

|  | Percent by Weight |
|---|---|
| n-butanol | 0.30 |
| 2-ethylhexanol | 9.09 |
| monobutylphthalate | 0.06 |
| mono-2-ethylhexylphthalate | 1.33 |
| dibutylphthalate | 4.93 |
| butyl-2-ethylhexylphthalate | 41.72 |
| di-2-ethylhexylphthalate | 42.38 |

The BOP/DOP weight ratio was 0.98 and the amount of dibutylphthalate as percent of total diester was 5.54.

### Example 4

A mixture of phthalic anhydride, n-butanol and 2-ethylhexanol was prepared in a 2-liter round bottom flask fitted with a heating mantle, temperature controller, stripping column, Stark & Dean trap, vacuum gauge, vacuum pump and variable speed agitator, by mixing 370.3 grams (2.5 moles) phthalic

4

anhydride with 157.5 grams (2.12 moles) butanol and 48.8 grams (0.37 moles) 2-ethylhexanol. The mixture was heated and maintained at 100°C for 1 hour. A second alcohol charge was then made consisting of 520.9 grams (4.0 moles) 2-ethylhexanol. Catalyst was then added (2 grams p-toluenesulfonic acid dissolved in 2 milliliters dissolved water) and the batch heated and maintained at 135°C for 6 hours under a total pressure ranging from 760 to 100 millimeters of mercury absolute. The resulting product analyzed as follows on a mole basis:

|  | Percent by Weight |
| --- | --- |
| n-butanol | 0.20 |
| 2-ethylhexanol | 8.56 |
| monobutylphthalate | 0.12 |
| mono-2-ethylhexylphthalate | 3.68 |
| dibutylphthalate | 3.07 |
| butyl-2-ethylhexylphthalate | 41.60 |
| di-2-ethylhexylphthalate | 42.57 |

The BOP/DOP weight ratio was 0.98 and the amount of dibutylphthalate as percent of total diester was 3.52.

Example 5

A mixture of phthalic anhydride, n-butanol and 2-ethylhexanol was prepared in a 2-liter round bottom flask fitted with a heating mantle, temperature controller, stripping column, Stark & Dean trap, vacuum gauge, vaccum pump and variable speed agitator, by mixing 370.3 grams (2.5 moles) phthalic anhydride with 157.5 grams (2.12 moles) butanol and 48.8 grams (0.37 moles) 2-ethylhexanol. The mixture was heated and maintained at 100°C for 1 hour. A second alcohol charge was then made consisting of 520.9 grams (4.0 moles) 2-ethylhexanol. Catalyst was then added (4 grams p-toluenesulfonic acid dissolved in 4 milliliters distilled water) and the batch heated and maintained at 135°C for 4.5 hours under a total pressure of 120 millimeters of mercury absolute. The resulting product analyzed as follows on a mole basis:

|  | Percent by Weight |
| --- | --- |
| n-butanol | 0.23 |
| 2-ethylhexanol | 10.90 |
| monobutylphthalate | 0.01 |
| mono-2-ethylhexylphthalate | 0.42 |
| dibutylphthalate | 2.20 |
| butyl-2-ethylhexylphthalate | 41.76 |
| di-2-ethylhexylphthalate | 44.08 |

The BOP/DOP weight ratio was 0.95 and the amount of dibutylphthalate as percent of total diester was 2.50.

Example 6

A mixture of phthalic anhydride and n-butanol was prepared in a 2-liter round bottom flask fitted with a heating mantle, temperature controller, stripping column, Stark & Dean trap, vacuum gauge, vaccum pump and variable speed agitator, by mixing 385.1 grams (2.6 moles) phthalic anhydride with 192.7 grams (2.6 moles) butanol. The mixture was heated and maintained at 100°C for 1 hour. A second alcohol charge was then made consisting of 474.0 grams (3.6 moles) 2-ethylhexanol. Catalyst was then added (4.2 grams p-toluenesulfonic acid dissolved in 4 milliliters distilled water) and the batch heated and maintained at 135°C for 5.5 hours under a total pressure ranging from 150 to 40 millimeters of mercury absolute. The resulting product analyzed as follows on a mole basis:

|  | Percent by Weight |
| --- | --- |
| n-butanol | 0.20 |
| 2-ethylhexanol | 5.78 |
| monobutylphthalate | 0.09 |
| mono-2-ethylhexylphthalate | 1.84 |
| dibutylphthalate | 5.59 |
| butyl-2-ethylhexylphthalate | 54.54 |
| di-2-ethylhexylphthalate | 31.55 |

The BOP/DOP weight ratio was 1.73 and the amount of dibutylphthalate as percent of total diester was 6.10.

Example 7

A mixture of phthalic anhydride and n-butanol was prepared in a 2-liter round bottom flask fitted with a heating mantle, temperature controller, stripping column, Stark & Dean trap, vacuum gauge, vaccum pump and variable speed agitator, by mixing 385.1 grams (2.6 moles) phthalic anhydride with 192.7 grams (2.6 moles) butanol. The mixture was heated and maintained at 100°C for 1 hour. A second alcohol charge was then made consisting of 474.0 grams (3.64 moles) 2-ethylhexanol. Catalyst was then added (4.2 grams p-toluenesulfonic acid dissolved in 4 milliliters distilled water) and the batch heated and maintained at 135°C for 3 hours under a total pressure ranging from 580 to 100 millimeters of mercury absolute. The overhead vapor was partially condensed at 90°C and the condensate was returned to the pot. The resulting product analyzed as follows on a mole basis:

|  | Percent by Weight |
| --- | --- |
| n-butanol | 0.00 |
| 2-ethylhexanol | 0.00 |
| monobutylphthalate | 0.06 |
| mono-2-ethylhexylphthalate | 5.90 |
| dibutylphthalate | 4.39 |
| butyl-2-ethylhexylphthalate | 53.60 |
| di-2-ethylhexylphthalate | 35.95 |

The BOP/DOP weight ratio was 1.49 and the amount of dibutylphthalate as percent of total diester was 4.68.

Example 8

A mixture of phthalic anhydride and n-butanol was prepared in a 2-liter round bottom flask fitted with a heating mantle, temperature controller, stripping column, Stark & Dean trap, vacuum gauge, vacuum pump and variable speed agitator, by mixing 385.1 grams (2.6 moles) phthalic anhydride with 192.7 grams (2.6 moles) butanol. The mixture was heated and maintained at 100°C for 1 hour. A second alcohol charge was then made consisting of 474.0 grams (3.64 moles) 2-ethylhexanol. Catalyst was then added (4.2 grams p-toluenesulfonic acid dissolved in 4 milliliters distilled water) and the batch heated and maintained at 130°C for 2 hours under a total pressure ranging from 360 to 20 millimeters of mercury absolute. The resulting product analyzed as follows on a mole basis:

| | Percent by Weight |
|---|---|
| n-butanol | 0.03 |
| 2-ethylhexanol | 1.95 |
| monobutylphthalate | 0.05 |
| mono-2-ethylhexylphthalate | 2.47 |
| dibutylphthalate | 3.73 |
| butyl-2-ethylhexylphthalate | 55.02 |
| di-2-ethylhexylphthalate | 36.34 |

The BOP/DOP weight ratio was 1.51 and the amount of dibutylphthalate as percent of total diester was 3.92.

Example 9

A mixture of phthalic anhydride and n-butanol was prepared in a 2-liter round bottom flask fitted with a heating mantle, temperature controller, stripping column, Stark & Dean trap, vacuum gauge, vacuum pump and variable speed agitator, by mixing 385.1 grams (2.6 moles) phthalic anhydride with 192.7 grams (2.6 moles) butanol. The mixture was heated and maintained at 100°C for 15 minutes. A second alcohol charge was then made consisting of 474.0 grams (3.64 moles) 2-ethylhexanol. Catalyst was then added (4.2 grams p-toluenesulfonic acid dissolved in 4 milliliters distilled water) and the batch heated and maintained at 130°C for 5.5 hours under a total pressure ranging from 360 to 20 millimeters of mercury absolute. The resulting product analyzed as follows on a mole basis:

| | Percent by Weight |
|---|---|
| n-butanol | 0.00 |
| 2-ethylhexanol | 0.78 |
| monobutylphthalate | 0.00 |
| mono-2-ethylhexylphthalate | 3.40 |
| dibutylphthalate | 5.39 |
| butyl-2-ethylhexylphthalate | 56.12 |
| di-2-ethylhexylphthalate | 33.91 |

The BOP/DOP weight ratio was 1.65 and the amount of dibutylphthalate as percent of total diester was 5.65.

Example 10

A mixture of phthalic anhydride and n-butanol was prepared in a 2-liter round bottom flask fitted with a heating mantle, temperature controller, stripping column, Stark & Dean trap, vacuum gauge, vacuum pump and variable speed agitator, by mixing 385.1 grams (2.6 moles) phthalic anhydride with 192.7 grams (2.6 moles) butanol. The mixture was heated and maintained at 110°C for 5 minutes. A second alcohol charge was then made consisting of 474.0 grams (3.64 moles) 2-ethylhexanol. Catalyst was then added (4.2 grams p-toluenesulfonic acid dissolved in 4 milliliters distilled water) and the batch heated and maintained at 130°C for 5 hours under a total pressure ranging from 760 to 140 millimeters of mercury absolute. The overhead vapor was partially condensed at 70°C and the condensate was returned to the pot. The resulting product analyzed as follows on a mole basis:

**0 039 878**

|  | Percent by Weight |
| --- | --- |
| n-butanol | 2.01 |
| 2-ethylhexanol | 3.51 |
| monobutylphthalate | 0.00 |
| mono-2-ethylhexylphthalate | 1.45 |
| dibutylphthalate | 6.53 |
| butyl-2-ethylhexylphthalate | 45.95 |
| di-2-ethylhexylphthalate | 42.15 |

The BOP/DOP weight ratio was 1.09 and the amount of dibutylphthalate as percent of total diester was 6.90.

Example 11

A mixture of phthalic anhydride, n-butanol and 2-ethylhexanol was prepared in a 2-liter round bottom flask fitted with a heating mantle, temperature controller, stripping column, Stark & Dean trap, vacuum gauge, vacuum pump and variable speed agitator, by mixing 370.3 grams (2.5 moles) phthalic anhydride with 157.5 grams (2.12 moles) butanol, and 48.8 grams (0.37 mole) 2-ethylhexanol. The mixture was heated and maintained at 95°C for 2 hours. A second alcohol charge was then made consisting of 520.9 grams (4.0 moles) 2-ethylhexanol. Catalyst was then added (2.0 grams p-toluenesulfonic acid dissolved in 2 milliliters distilled water) and the batch heated and maintained at 125°C for 1 hour under a total pressure ranging from 430 to 250 millimeters of mercury absolute. None of the overhead vapor was returned to the pot as condensate. The batch was then heated and maintained at 125°C for an additional 4.5 hours under total pressure of 50 to 100 millimeters mercury. The resulting product analyzed as follows on a mole basis:

|  | Percent by Weight |
| --- | --- |
| n-butanol | 0.20 |
| 2-ethylhexanol | 10.44 |
| monobutylphthalate | 0.11 |
| mono-2-ethylhexylphthalate | 3.94 |
| dibutylphthalate | 2.33 |
| butyl-2-ethylhexylphthalate | 42.91 |
| di-2-ethylhexylphthalate | 39.86 |

The BOP/DOP weight ratio was 1.08 and the amount of dibutylphthalate as percent of total diester was 2.73.

**Claims**

1. A method of mixing a mixed ester plasticizer for vinyl plastics and the like comprising the steps of first reacting an alcohol composition consisting of 100 to 80 mole percent butyl alcohol and 0 to 20 mole percent octyl alcohol with phthalic anhydride in a mole ratio of about 0.80 to about 1.5 moles of alcohol total per mole of phthalic anhydride, adding an octyl alcohol in a stoichiometric amount based on unreacted acid groups along with 20 to 100 percent of the stoichiometric amount of octyl alcohol as excess, adding an esterification catalyst and heating the reaction mixture at a maximum temperature of about 150°C for a time period sufficient to carry the esterification to substantial completion.

2. The process of claim 1 wherein said alcohol phthalic anhydride reaction is at a temperature of about 80 to 120°C for a period of time of about 0.25 to 3 hours and the subsequent reaction with said octyl alcohol is at a temperature of about 120 to 140°C for a time of from about 1 to 10 hours.

3. The method of claim 2 wherein said butyl alcohol is n-butyl alcohol and said octyl alcohol is 2-ethylhexyl alcohol.

8

4. The method of claim 2 wherein said esterification catalyst is p-toluene sulfonic acid.

5. The method of claim 2 which includes the additional steps of neutralizing the unreacted acid with an aqueous alkaline solution capable of converting the acid to water-soluble salts, and purifying the resultant ester mixture by the removal of salt solution, excess alkaline solution, excess alcohol and catalyst.

6. The method of claim 5 wherein said butyl alcohol is n-butyl alcohol and said octyl alcohol is 2-ethylhexyl alcohol.

7. The method of claim 6 wherein said esterification catalyst is p-toluene sulfonic acid.

## Patentansprüche

1. Verfahren zur Herstellung eines Mischester-Weichmachers für Vinylkunststoffe und dergleichen, dadurch gekennzeichnet, daß man zunächst ein Alkoholgemisch aus 100 bis 80 Mol% Butylalkohol und 0 bis 20 Mol% Oktylalkohol mit Phthalsäure in einem Molverhältnis von etwa 0,80 bis etwa 1,5 Mol Alkohol je Mol Phthalsäure umsetzt, dann einen Oktylalkohol in stöchiometrischer Menge, bezogen auf nicht umgesetzte Säuregruppen, zusammen mit 20 bis 100% der stöchiometrischen Menge als Überschuß zusetzt, einen Veresterungskatalysator zufügt und das Reaktionsgemisch so lange bei einer Temperatur von etwa 150°C erhitzt, bis die Veresterung im wesentlichen vollständig ist.

2. Verfahren nach Anspruch 1, dadurch gekennzeichnet, daß man die Alkohol-Phthalsäure-Umsetzung etwa 0,25 bis 3 Stunden bei einer Temperatur von etwa 80 bis 120°C und die anschließende Umsetzung mit dem Oktylalkohol etwa 1 bis 10 Stunden bei einer Temperatur von etwa 120 bis 140°C durchführt.

3. Verfahren nach Anspruch 2, dadurch gekennzeichnet, daß man als Butylalkohol n-Butylalkohol und als Oktylalkohol 2-Ethylhexylalkohol verwendet.

4. Verfahren nach Anspruch 2, dadurch gekennzeichnet, daß man als Veresterungskatalysator p-Toluolsulfonsäure verwendet.

5. Verfahren nach Anspruch 2, dadurch gekennzeichnet, daß man zusätzlich die nicht umgesetzte Säure mit einer wäßrigen alkalischen Lösung, die die Säure in wasserlösliche Salze umzuwandeln vermag, neutralisiert und die erhaltene Estermischung durch Abtrennen der Salzlösung, der überschüssigen alkalischen Lösung, des überschüssigen Alkohols und des Katalysators reinigt.

6. Verfahren nach Anspruch 5, dadurch gekennzeichnet, daß man als Butylalkohol n-Butylalkohol und als Oktylalkohol 2-Ethylhexylalkohol verwendet.

7. Verfahren nach Anspruch 6, dadurch gekennzeichnet, daß man als Veresterungskatalysator p-Toluolsulfonsäure verwendet.

## Revendications

1. Procédé de préparation d'un plastifiant ester mixte pour des matières plastiques vinyliques et analogues, selon lequel on fait d'abord réagir une composition alcoolique, constituée de 100 à 80 moles % d'alcool butylique et 0 à 20 moles % d'alcool octylique, avec de l'anhydride phtalique en rapport molaire d'environ 0,80 à environ 1,5 mole d'alcool total par mole d'anhydride phtalique, on ajoute un alcool octylique en quantité stoechiométrique, basée sur des groupes acide n'ayant pas réagi, en même temps qu'avec 20 à 100% de la quantité stoechiométrique d'alcool octylique en excès, on ajoute un catalyseur d'estérification et on chauffe le mélange de réaction à une température maximale d'environ 150°C pendant un temps suffisant pour amener l'estérification sensiblement à sa fin.

2. Procédé selon la revendication 1, dans lequel la réaction alcool-anhydride phtalique s'effectue à une température d'environ 80 à 120°C pendant une durée d'environ 0,25 à 3 heures et la réaction suivante avec ledit alcool octylique s'effectue à une température d'environ 120 à 140°C pendant une durée allant d'environ 1 à 10 heures.

3. Procédé selon la revendication 2, dans lequel ledit alcool butylique est l'alcool n-butylique et ledit alcool octylique est l'alcool 2-éthylhexylique.

4. Procédé selon la revendication 2, dans lequel ledit catalyseur d'estérification est l'acide p-toluènesulfonique.

5. Procédé selon la revendication 2 qui comprend les stades additionnels dans lesquels on neutralise l'acide n'ayant pas réagi avec une solution alcaline aqueuse capable de convertir l'acide en sels solubles dans l'eau, et on purifie le mélange d'ester résultant, en éliminant la solution de sel, la solution alcaline en excès, l'alcool en excès et le catalyseur.

6. Procédé selon la revendication 5, dans lequel ledit alcool butylique est l'alcool n-butylique et ledit alcool octylique est l'alcool 2-éthylhexylique.

7. Procédé selon la revendication 6, dans lequel le catalyseur d'estérification est l'acide p-toluènesulfonique.